(19) Europäisches Patentamt
European Patent Office
Office européen des brevets

(11) EP 3 122 429 B1

# (12) EUROPEAN PATENT SPECIFICATION

(45) Date of publication and mention of the grant of the patent:
**09.05.2018 Bulletin 2018/19**

(21) Application number: **15715922.9**

(22) Date of filing: **25.03.2015**

(51) Int Cl.:
*A61Q 19/04* (2006.01) *A61Q 5/10* (2006.01)
*A61Q 5/08* (2006.01) *A61Q 5/04* (2006.01)
*C09D 5/00* (2006.01) *C09D 143/04* (2006.01)
*C09D 183/00* (2006.01) *A61K 8/25* (2006.01)
*A61K 8/58* (2006.01)

(86) International application number:
**PCT/US2015/022381**

(87) International publication number:
**WO 2015/148603 (01.10.2015 Gazette 2015/39)**

## (54) SILYLATED SURFACTANTS IN PERSONAL CARE AND HOME CARE

SILYLIERTE TENSIDE IN KÖRPERPFLEGE- UND HAUSPFLEGEPRODUKTEN

TENSIOACTIFS SILYLÉS EN PRÉPARATIONS DE SOINS PERSONNELS ET DE SOINS À MÉNAGE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.03.2014 US 201461970663 P**

(43) Date of publication of application:
**01.02.2017 Bulletin 2017/05**

(73) Proprietor: **Momentive Performance Materials Inc.**
**Waterford, NY 12188 (US)**

(72) Inventors:
- **FALK, Benjamin**
  **Yorktown Heights, NY 10598 (US)**
- **RINARD, Chauncey, J.**
  **Newport, OH 45678 (US)**
- **DUSSAUD, Ann, Dominique**
  **Tarrytown, NY 10591 (US)**
- **PRESTI, Richard, Anthony**
  **Airmont, NY 10952 (US)**
- **SPERRING, Susan, M.**
  **Pomona, NY 10970 (US)**

(74) Representative: **Zinnecker, Armin**
**Lorenz Seidler Gossel**
**Rechtsanwälte Patentanwälte**
**Partnerschaft mbB**
**Widenmayerstraße 23**
**80538 München (DE)**

(56) References cited:
**WO-A1-2007/127016** **WO-A1-2010/099173**
**JP-A- 2011 116 665** **US-A- 4 034 064**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 122 429 B1

## Description

FIELD OF THE INVENTION

The present invention relates to organomodified silylated surfactant compositions that exhibit resistance to hydrolysis over a wide pH range in combination with and oxidation or reducing agent effective for use in personal care and home care applications. More particularly the present invention relates to such hydrolysis-resistant organomodified silylated surfactants having a resistance to hydrolysis between a pH of about 2 to a pH of about 12. More particularly the present invention relates to the use of such hydrolysis resistant organomodified silylated surfactants in cosmetic compositions and home care compositions containing peroxides, in the presence of less than 2 ppm catalyst. Surfactants of the specific type used herein are disclosed in e.g. WO 2007/127016 A1 (MOMENTIVE PERFORMANCE MAT INC [US]; LEATHERMAN MARK D [US]; PENG WENQI) 8 November 2007 (2007-11-08)

BACKGROUND OF THE INVENTION

The topical application of liquid compositions to surfaces to effect a desired change involve the processes of controlling wetting, spreading, foaming, penetrating, detergency, and the like. When used in aqueous solutions to improve the delivery of active ingredients to the surface being treated, trisiloxane-type compounds have been found to be useful in enabling the control of these processes to achieve the desired effect. However, the trisiloxane compounds may only be used in a narrow pH range, ranging from a slightly acidic pH of 6 to a very mildly basic pH of 7.5. Outside this narrow pH range, the trisiloxane compounds are not stable to hydrolysis, undergoing rapid decomposition. Traditional silicon-based surfactants are synthesized using a homogeneous hydrosilylation precious metal catalyst. These catalysts are left in the product. The hydrosilylation catalysts or their decomposition products typically undesirably catalyze the decomposition of peroxide containing formulations. Silicon based surfactants that are stable to hydrolysis over a wide pH range and do not contain significant levels of residual hydrosilylation catalyst are very desirable, as such compounds would allow more effective formulations regarding the topical application of liquid compositions to surfaces. Such compositions may result in increased effectiveness, reduction of irritation, increased and move even coverage, or the use of smaller quantities of actives or formulation.

SUMMARY OF THE INVENTION

The present invention provides for personal care, e.g., cosmetic compositions, and homecare compositions that contain peroxides, utilizing an organomodified silylated surfactant compound or compositions thereof, useful as a surfactant, wherein the organomodified silylated surfactant compound has the general formula:

$$(R^1)(R^2)(R^3)Si\text{-}R^4\text{-}Si(R^5)(R^6)(R^7)$$

wherein

$R^1$, $R^2$, $R^3$, $R^5$, and $R^6$ are each independently selected from the group consisting of 1 to 6 monovalent hydrocarbon radicals, aryl, and a hydrocarbon group of 7 to 10 carbons containing an aryl group;
$R^4$ is a hydrocarbon group of 1 to 3 carbons.
$R^7$ is an alkyleneoxide group of the general formula:

$$R^8(C_2H_4O)_d(C_3H_6O)_e(C_4H_8O)_fR^9$$

where $R^8$ is a divalent linear or branched hydrocarbon radical having the structure:

$$\text{-}CH_2\text{-}CH(R^{10})(R^{11})_gO\text{-}$$

where $R^{10}$ is H or methyl; $R^{11}$ is a divalent alkyl radical of 1 to 6 carbons where the subscript g may be 0 or 1;
$R^9$ is selected from the group consisting of H, monovalent hydrocarbon radicals of 1 to 6 carbon atoms and acetyl, subject to the limitation that the subscripts e and f are zero or positive and satisfy the following relationships:

$$2 \leq d + e + f \leq 20 \text{ with } d \geq 2.$$

These composition(s) contain less than 2 ppm of catalyst as described herein.

DETAILED DESCRIPTION OF THE INVENTION

As used herein, integer values of stoichiometric subscripts refer to molecular species and non-integer values of stoichiometric subscripts refer to a mixture of molecular species on a molecular weight average basis, a number average basis or a mole fraction basis.

The present invention provides for personal care, e.g., cosmetic, and homecare compositions containing peroxides, sunless tanners, oxidizing agents, or reducing agents utilizing an organomodified silylated surfactant compound or compositions thereof useful as a surfactant wherein the organomodified silylated surfactant compound has the general formula:

$$(R^1)(R^2)(R^3)Si\text{-}R^4\text{-}Si(R^5)(R^6)(R^7)$$

wherein

$R^1$, $R^2$, $R^3$, $R^5$, and $R^6$ are each independently selected from the group consisting of 1 to 6 carbon atom monovalent hydrocarbon radicals, 6 to 10 carbon atom aryl groups, and a monovalent hydrocarbon group of 7 to 10 carbon atoms containing an aryl group;
$R^4$ is a hydrocarbon group of 1 to 3 carbons;
$R^7$ is an alkyleneoxide group of the general formula:

$$R^8(C_2H_4O)_d(C_3H_6O)_e(C_4H_8O)_fR^9$$

where $R^8$ is a divalent linear or branched hydrocarbon radical having the structure:

$$\text{-}CH_2\text{-}CH(R^{10})(R^{11})_gO\text{-}$$

where $R^{10}$ is H or methyl; $R^{11}$ is a divalent alkyl radical of 1 to 6 carbons where the subscript g may be 0 or 1; $R^9$ is selected from the group consisting of H, monovalent hydrocarbon radicals of 1 to 6 carbon atoms and acetyl subject to the limitation that the subscripts e and f are zero or positive and satisfy the following relationships:

$$2 \leq d + e + f \leq 20 \text{ with } d \geq 2.$$

While not wishing to be bound by theory, it is believed that an amount of less than 2 ppm of catalyst present in the subject composition provides for personal care and home care compositions, as described herein, wherein the the organomodified silylated surfactant compound having the general formula:

$$(R^1)(R^2)(R^3)Si\text{-}R^4\text{-}Si(R^5)(R^6)(R^7)$$

as is described herein can be used with a personal care active component or a home care component, respectively, wherein said composition has an enhanced resistance to hydrolysis and stability as described herein without compromising the shelf life of the personal care active component or home care component. The presence of catalyst in excess of the low amount employed herein are believed to undesirably catalyze the decomposition of peroxide containing formulations which has been unexpectedly herein discovered for the first time.

Preferred embodiments

One preferred embodiment of the organomodified silylated surfactant is where $R^1$, $R^2$, R3, $R^5$, and $R^6$ are each independently selected from the group consisting of 1 to 6 monovalent hydrocarbon radicals, aryl, and a hydrocarbon group of 7 to 10 carbons containing or substituted with an aryl group; preferably a hydrocarbon group of 7 to 8 carbons containing an aryl group and more preferably a hydrocarbon group of 7 carbons containing an aryl group;
$R^4$ is a hydrocarbon group of 1 to 3 carbons;
$R^7$ is an alkyleneoxide group of the general formula:

$$R^8(C_2H_4O)d(C_3H_6O)_e(C_4H_8O)_fR^9$$

where $R^8$ is a divalent linear or branched hydrocarbon radical having the structure:

$$-CH_2-CH(R^{10})(R^{11})_gO-$$

$R^{10}$ is H or methyl; $R^{11}$ is a divalent alkyl radical of 1 to 6 carbon atoms, more preferably 1 to 2 carbon atoms, where the subscript g may be 0 or 1;
$R^9$ is selected from the group consisting of H, monovalent hydrocarbon radicals of 1 to 6 carbon atoms and acetyl; more preferably H or methyl, where $R^7$ is subject to the limitation that the subscripts d, e and f are zero or positive and satisfy the following relationships:

$$2 \leq d + e + f \leq 20 \text{ with } d \geq 2;$$

with d preferably ranging from 3 to 20, and more preferably from 5 to 8; with e preferably ranging from 0 to 10; and more preferably 0 to 5; with f preferably ranging from 0 to 8, and more preferably from 0 to 4.

Another preferred embodiment of the organomodified silylated surfactant is where $R^1$, $R^2$, $R^3$, $R^5$, and $R^6$ are each methyl; $R^4$ is a hydrocarbon group of 1 to 3 carbon atoms; $R^{10}$ is H; $R^{11}$ is methyl, where the subscript g is 1. $R^9$ is H or methyl. Subscripts e and f are zero or positive and satisfy the following relationships:

$$2 \leq d + e + f \leq 17 \text{ with } d \geq 2;$$

preferably d ranges from 3 to 9, and more preferably from 5 to 8; preferably e ranges from 0 to 5; and more preferably 0 to 3; preferably f is 0 to 2. Yet another preferred embodiment of the organomodified silylated surfactant is where $R^1$, $R^2$, $R^3$, $R^5$, and $R^6$ are each methyl; $R^4$ is a hydrocarbon group of 1 or 2 carbon atoms;
$R^{10}$ is H; $R^{11}$ is methyl, where the subscript g is 1; the subscript d ranges from 6 to 8, both e and f are 0.

One method of producing the composition of the present invention is to react a molecule of the following formula:

$$(R^1)(R^2)(R^3)Si-R^4-Si(R^5)(R^6)(R^{12})$$

where $R^{12}$ is H, wherein the definitions and relationships are later defined and consistent with those defined above, under hydrosilylation conditions and in the presence of a catalyst, with an olefinically modified polyalkyleneoxide, such as allyloxypolyethyleneglycol, or methallyloxypolyalkyleneoxide, which are incorporated here as examples, and not set forth to limit other possible olefinically modified alkyleneoxide components. As used herein the phrase "olefinically modified polyalkyleneoxide" is defined as a molecule possessing one or more alkyleneoxide groups containing one or more, terminal or pendant, carbon-carbon double bonds. The polyether is an olefinically modified polyalkyleneoxide (hereinafter referred to as "polyether") is described by the general formula :

$$CH_2=CH(R^{10})(R^{11})_gO(C_2H_4O)_d(C_3H_6O)_e(C_4H_8O)_fR^9$$

where
$R^{10}$ is H or methyl; $R^{11}$ is a divalent alkyl radical of 1 to 6 carbons where the subscript g may be 0 or 1; $R^9$ is H, a monofunctional hydrocarbon radical of 1 to 6 carbons, or acetyl. When the polyether is composed of mixed oxyalkyleneoxide groups (i.e. oxyethylene, oxypropylene and oxybutylene) the units may be blocked, or randomly distributed. Illustrative examples of blocked configurations are: -(oxyethylene)$_a$(oxypropylene)$_b$-; -(oxybutylene)$_c$(oxyethylene)$_a$-; -(oxypropylene)$_b$(oxyethylene)$_a$(oxybutylene)$_c$-, and the like.

Non-limiting illustrative examples of the olefinically modified polyalkyleneoxide are:

$CH_2=CHCH_2O(CH_2CH_2O)_8H$; $CH_2=CHCH_2O(CH_2CH_2O)_8CH_3$;

$CH_2=CHCH_2O(CH_2CH_2O)_4(CH_2CH(CH_3)O)_5H$;

$CH_2=CHO(CH_2CH_2O)_5(CH_2CH(CH_3)O)_5H$;

$CH_2=C(CH_3)CH_2O(CH_2CH_2O)_4(CH_2CH(CH_3)O)_5C(=O)CH_3$;

$CH_2=CHCH_2O(CH_2CH_2O)_5(CH_2CH(CH_3)O)_2(CH_2CH(CH_2CH_3)O)_2H$

Polyether-modified carbosilanes are straightforwardly prepared through the use of a hydrosilylation reaction to graft the olefinically modified (i.e. vinyl, allyl or methallyl) polyalkyleneoxide onto the hydride (SiH) intermediate of the trisiloxane of the present invention.

Precious metal catalysts suitable for making polyether-substituted silanes are also well known in the art and comprise complexes of rhodium, ruthenium, palladium, osmium, iridium, and / or platinum. Many types of platinum catalysts for this SiH-olefin addition reaction are known and such platinum catalysts may be used to generate the compositions of the present invention. The platinum compound can be selected from those having the formula ($PtCl_2$Olefin) and H($PtCl_3$Olefin) as described in U.S. Pat. No. 3159601 . A further platinum containing material can be a complex of chloroplatinic acid with up to 2 moles per gram of platinum of a member selected from the class consisting of alcohols, ethers, aldehydes and mixtures thereof as described in U.S. Pat. No. 3,220,972. Yet another group of platinum containing materials useful in this present invention is described in U.S. Pat. Nos. 3,715,334; 3,775,452 and 3,814,730 (Karstedt). Those skilled in the art can easily determine an effective amount of catalyst to employ herein, e.g., a platinum catalyst. Generally an effective amount of catalyst is such as the "low content" amounts described herein such as the non-limiting embodiments of ranges from about 0.1 to 2 parts per million (ppm) of the total organomodified silylated surfactant composition (or silicon-containing compound), more specifically 0.1 to about 1 ppm of the total organomodified silylated surfactant composition (or silicon-containing compound) and most specifically from about 0.1 to about 0.5 ppm of the total organomodified silylated surfactant composition (or silicon-containing compound). These ranges of amounts of catalyst can also be used for the silicon-containing compound in the personal care composition, home care compositions and low catalyst content silicon containing compound composition. Heterogeneous catalysts which are essentially employed herein are such as those described in U.S. Pat. 8,497,338 B2 which discloses the use of Pt, Pd, Rh, Ru, Ir and Os loaded on an organic polymeric support or an inorganic support selected from the group consisting of activated carbon, functionalized or non-functionalized silica, alumina, zeolite, and nanoparticles. If a heterogeneous catalyst is employed in the synthesis of the organomodified silylated surfactant then the residual catalyst must be removed ether through a chemical reaction or a complexation followed by filtration.

The compositions of the present invention exhibit an enhanced resistance to hydrolysis outside a pH range ranging from 6 to 7.5. Enhanced resistance to hydrolysis can be demonstrated by a variety of tests but as used herein enhanced resistance to hydrolysis means 50 mole percent or more of the hydrolysis resistant composition of the present invention remains unchanged or unreacted after a period of a twenty-four exposure to aqueous acidic conditions where the solution has a pH lower than 6 or after a period of a twenty-four hour exposure to aqueous basic conditions where the solution has a pH greater than 7.5. Under acidic conditions the compositions of the present invention show a survival of 50 mole percent of the original concentration or greater at a pH of 5 or less for a period of time in excess of 48 hours; specifically the compositions of the present invention show a survival of 50 mole percent or greater at a pH of 5 or less for a period of time in excess of 2 weeks; more specifically the compositions of the present invention show a survival of 50 mole percent or greater at a pH of 5 or less for a period of time in excess of 1 month; and most specifically the compositions of the present invention show a survival of 50 mole percent or greater at a pH of 5 or less for a period of time in excess of 6 months. Under basic conditions the compositions of the present invention show a survival of 50 mole percent or greater at a pH of 8 or more for a period of time in excess of 2 weeks; specifically the compositions of the present invention show a survival of 50 mole percent or greater at a pH of 8 or more for a period of time in excess of 4 weeks; more specifically the compositions of the present invention show a survival of 50 mole percent or greater at a pH of 8 or more for a period of time in excess of 6 months; and most specifically the compositions of the present invention show a survival of 50 mole percent or greater at a pH of 8 or more for a period of time in excess of 1 year.

Additionally the composition of the present invention will not accelerate the decomposition of peroxide containing formulations.

The peroxides employed in the invention herein may comprise those selected from the group consisting of hydrogen peroxide, ammonium persulfate, potassium persulfate, diisopropyl peroxide, dilauryl peroxide; di-t-butyl peroxide, dicumyl peroxide, t-butyl hydrogen peroxide, t-amyl hydrogen peroxide, cumyl hydrogen peroxide, acetyl peroxide, lauroyl peroxide, benzoyl peroxide, ethyl peroxybenzoate and pavalate peroxide, as well as peroxides of, for instance, lipids, proteins, carbohydrates and free radicals derived from the same.

For purposes of the present patent specification, the terms "sunless tanning active or agent," "sunless tanner," "skin tanner," "skin tanning active or agent" and "self tanning agent" can be used interchangeably. A sunless tanner refers to any compound or material which imparts an enzymatic reaction which results in a tan or brown appearance to the skin, without the need to expose the skin to the sun or other radiant energy sources. Representative sunless tanners include, but are not limited to dihydroxyacetone (DHA), erythrulose, and botanical extracts of plants such as the silver birch (Betulla alba), or Eclipta alba which contains flavonoids known as wedelolactone, demethylwedelolactone and mixtures thereof.

Oxidizing agents that may be employed herein include those such as potassium bromate, persulfates such as ammonium persulfate, potassium persulfate and sodium persulfate, ozone, hydrogen peroxide, or a peracid such as

periodate, dinitrogen tetroxide, dimethol sulfoxide/acetic anhydride, gaseous oxygen, hypochlorite, hypobromite, chromic acid and chromates, hypochlorous acid, hypobromous acid, hypoiodous acid, perborates, perphosphates, oxidizing metal compounds, nitroxy compounds, urea peroxide, alkaline metal bromates, polythonates and persalts such as perboratexs, percarbonates and persulfates, and enzymes such as peroxydases and two-electron oxydo-deuctases. and combinations thereof, and the like.

Reducing agents that may be employed herein are thiols such as those chosen from cysteine and derivatives thereof, i.e. n-acetylcysteine, cysteamine, and derivatives therof, for instance $C_1$-$C_4$ acylated derivatives such as N-acetyl cysteamine and N-propionyl cysteamine, thiolactic acid and esters thereof, such as glycerol monothiolactate, thioglycolic acid and esters thereof, such as glycerol monothioglycolate,and thioglycerol and salts thereof, as well as other known reducing agents.

The amount of peroxides, sunless tanning agent, oxidizing agent and reducing agent that may be employed will depend on what application each of these components is directed to and the nature of the other components of the application, but in general the amount of each of these components employed in the applications herein can be from about 0.1 weight percent to about 10 weight percent, more specifically from about 1 to about 7 weight percent based on the weight of the personal care or home care composition.

USES FOR THE COMPOSITIONS OF THE PRESENT INVENTION:

A. Personal Care

In a preferred embodiment, the organomodified silylated surfactant of the present invention comprises, per 100 parts by weight ("pbw") of the personal care composition, from 0.01 to 99 pbw, more preferably from 0.05 pbw to 30 pbw and still more preferably from 0.1 to 15 pbw of the organomodified silylated surfactant.

The organomodified silylated surfactant compositions of the present invention may be utilized in personal care emulsions, such as lotions, and creams. As is generally known, emulsions comprise at least two immiscible phases, one of which is continuous and the other discontinuous. Further, emulsions may be liquids with varying viscosities or solids. Additionally the particle size of the emulsions may render them microemulsions, and when the particle sizes are sufficiently small, microemulsions may be transparent. Further, it is also possible to prepare emulsions of emulsions and these are generally known as multiple emulsions. These emulsions may be:

1) aqueous emulsions where the discontinuous phase comprises water and the continuous phase comprises the organomodified silylated surfactant of the present invention;

2) aqueous emulsions where the discontinuous phase comprises the organomodified silylated surfactant of the present invention and the continuous phase comprises water;

3) non-aqueous emulsions where the discontinuous phase comprises a non-aqueous hydroxylic solvent and the continuous phase comprises the organomodified silylated surfactant of the present invention; and

4) non-aqueous emulsions where the discontinuous phase comprises the organomodified silylated surfactant of the present invention and the continuous phase comprises a non-aqueous hydroxylic organic solvent.

Non-aqueous emulsions comprising a silicone phase are described in US patent 6,060,546 and US patent 6271295. As used herein, the term "non-aqueous hydroxylic organic compound" means hydroxyl-containing organic compounds exemplified by alcohols, glycols, polyhydric alcohols and polymeric glycols, and mixtures thereof that are liquid at room temperature, e.g. about 25 °C, and about one atmosphere pressure. The non-aqueous organic hydroxylic solvents are selected from the group consisting of hydroxyl-containing organic compounds comprising alcohols, glycols, polyhydric alcohols and polymeric glycols, and mixtures thereof that are liquid at room temperature, e.g. about 25 °C, and about one atmosphere pressure. Preferably the non-aqueous hydroxylic organic solvent is selected from the group consisting of ethylene glycol, ethanol, propyl alcohol, iso-propyl alcohol, propylene glycol, dipropylene glycol, tripropylene glycol, butylene glycol, iso-butylene glycol, methyl propane diol, glycerin, sorbitol, polyethylene glycol, polypropylene glycol mono alkyl ethers, polyoxyalkylene copolymers and mixtures thereof.

Once the desired form is attained whether as a silicone only phase, an anhydrous mixture comprising the silicone phase, a hydrous mixture comprising the silicone phase, a water-in-oil emulsion, an oil-in-water emulsion, or either of the two non-aqueous emulsions or variations thereon, the resulting material is usually a cream or lotion with improved deposition properties and good feel characteristics. It is capable of being blended into formulations for hair care, skin care, antiperspirants, sunscreens, cosmetics, color cosmetics, insect repellants, vitamin, steroid and hormone carriers, fragrance carriers and the like.

The personal care applications where the organomodified silylated surfactant of the present invention and the silicone compositions derived therefrom of the present invention may be employed include, but are not limited to, deodorants, antiperspirants, antiperspirant/deodorants, shaving products, skin lotions, moisturizers, toners, facial masks, bath products, cleansing products,, feminine hygiene products, hair care products such as shampoos, conditioners, mousses, styling gels, hair sprays, hair dyes, hair color products, hair bleaches, permanents, hair straighteners, manicure products such as nail polish, nail polish remover, nails creams and lotions, cuticle softeners, protective creams such as sunscreen, insect repellent and anti-aging products, color cosmetics such as lipsticks, foundations, face powders, eye liners, eye shadows, blushes, makeup, mascaras, Sunless tanners, depilatory, hair and skin bleaching bleachers, anti-acne treatments, wipes, oral rinses, fluorinated toothpastes and gels, topical ointments (including anti-inflammatory, anti-fungal and, anti-itch), shave preparations, hair lighteners, make-up stains and other personal care formulations where silicone components have been conventionally added, as well as drug delivery systems for topical application of medicinal compositions that are to be applied to the skin.

In a preferred embodiment, the personal care composition of the present invention further comprises one or more personal care ingredients. Suitable personal care ingredients include, for example, emollients, moisturizers, humectants, pigments, including pearlescent pigments such as, for example, bismuth oxychloride and titanium dioxide coated mica, colorants, fragrances, biocides, preservatives, antioxidants, antimicrobial agents, anti-fungal agents, antiperspirant agents, antidandruff agents, materials for treating the scalp such as topical hair growth drugs (minoxidil), hair growth inhibiting material (5-alpha reductase inhibitors) dandruff control, exfoliants, hormones, enzymes, steroids, peptides, proteins, medicinal compounds, vitamins, salts, electrolytes, alcohols, polyols, absorbing agents for ultraviolet radiation, botanical extracts, nonionic surfactants, cationic surfactants, silicone surfactants, anionic surfactants, zwitterionic (amphoteric) surfactants, silicone oils, organic oils, waxes, film formers, thickening agents such as, for example, acrylic based thickeners, natural product thickeners such as guar or modified guar, fumed silica or hydrated silica, particulate fillers, such as for example, talc, kaolin, starch, modified starch, mica, nylon, clays, such as, for example, bentonite and organo-modified clays.

Suitable personal care compositions are made by combining, in a manner known in the art, such as, for example, by mixing, one or more of the above components with the organomodified silylated surfactant. Suitable personal care compositions may be in the form of a single phase or in the form of an emulsion, including oil-in-water, water-in-oil and anhydrous emulsions where the silicone phase may be either the discontinuous phase or the continuous phase, as well as multiple emulsions, such as, for example, oil-in water-in-oil emulsions and water-in-oil-in water-emulsions.

In one useful embodiment, an antiperspirant composition comprises the organomodified silylated surfactant of the present invention and one or more active antiperspirant agents. Suitable antiperspirant agents include, the non-limiting examples of, aluminum halides, aluminum hydroxyhalides, for example, aluminum chlorohydrate, and complexes or mixtures thereof with zirconyl oxyhalides and zirconyl hydroxyhalides, such as for example, aluminum-zirconium chlorohydrate, aluminum zirconium glycine complexes, such as, for example, aluminum zirconium tetrachlorohydrex gly.

In another useful embodiment, a skin care composition comprises the organomodified silylated surfactant, and a vehicle, such as, for example, a silicone oil or an organic oil. The skin care composition may, optionally, further include emollients, such as, for example, triglyceride esters, wax esters, alkyl or alkenyl esters of fatty acids or polyhydric alcohol esters and one or more the known components conventionally used in skin care compositions, such as, for example, pigments, vitamins, such as, for example, Vitamin A, Vitamin C and Vitamin E, sunscreen or sunblock compounds, such as, for example, titanium dioxide, zinc oxide, oxybenzone, octylmethoxy cinnamate, butylmethoxy dibenzoyl methane, p-aminobenzoic acid and octyl dimethyl-p-aminobenzoic acid short chain peptides, proteins, skin lightening agents and humectants (i.e. hyaluronic acid and other GAGs), heating agents, cooling agents, barrier repair materials (ceramides, cholesterol, etc).

In another useful embodiment, a color cosmetic composition, such as, for example, a lipstick, a makeup or a mascara composition comprises the organomodified silylated surfactant, and a coloring agent, such as a pigment, a water soluble dye or a liposoluble dye.

In another useful embodiment, the compositions of the present invention are utilized in conjunction with fragrant materials. These fragrant materials may be fragrant compounds, encapsulated fragrant compounds, or fragrance releasing compounds that either the neat compounds or are encapsulated. Particularly compatible with the compositions of the present invention are the fragrance-releasing silicon-containing compounds as disclosed in US patents 6,046,156; 6,054,547; 6,075,111; 6,077,923; 6,083,901; and 6153578.

B. Home Care

Compositions of the present organomodified silylated surfactant invention are useful in home care applications, including laundry detergent and fabric softener, dishwashing liquids, wood and furniture polish, floor polish, tub and tile cleaners, toilet bowl cleaners, hard surface cleaners, window cleaners, antifog agents, drain cleaners, auto-dishwashing detergents and sheeting agents, carpet cleaners, prewash spotters, rust cleaners and scale removers. The amount of

organomodified silylated surfactant that can be employed in the home care composition can vary greatly depending on the particular home care application but in one non-limiting embodiment can be the same amounts as described above for the personal care composition.

EXPERIMENTAL

The hydride intermediates for the organomodified silylated surfactant compositions of the present invention, as well as comparative compositions were prepared as described in the following examples.

Preparation Example 1

Hydrosilylation of 1-(2-trimethylsilylethyl)-1,1-dimethylsilane with a methyl capped allylpolyethyleneglycol, Catalysis with 5% Platinum on Alumina (1.35 Moles of APEG400 to 1 Mole carbodisilane)

To a 4-necked, 500 mL round bottom flask, equipped with an overhead stirrer, Friedrich condenser, temperature sensor (thermocouple connected to a temperature controller), heating mantle and nitrogen purge tube were charged 380.9 grams (0.952 moles) of a methyl capped allylpolyethyleneglycol (MW ~ 400 g/mole), 119.3 grams (94.6% purity, 0.704 moles) of 1-(2-trimethylsilylethyl)-1,1-dimethylsilane and 0.2 grams of 5% platinum on alumina (20 ppm Pt based on total flask charge). Under a nitrogen blanket, agitation was initiated and the temperature of the flask contents slowly brought to 130°C. The reaction mixture was allowed to stir for 18 hours (overnight) at 130°C. After 18 hours (overnight), 0.1 cc$H_2$/g of silanic hydrogen was detected in the product. The Friedrich condenser was removed and the flask was fitted with a cold-finger condenser and a receiver and a nitrogen sparge was initiated. After 60 minutes, the product was permitted to cool to room temperature and subsequently filtered under nitrogen pressure through a stainless steel pressure filter fitted with a 5-micron filter pad to yield a clear, colorless fluid (Structure 1).

Structure 1

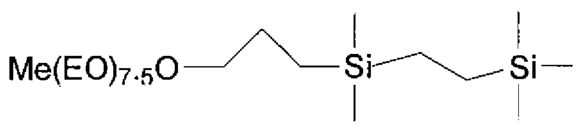

Preparation Example 1b

The product of Preparation Example 1 (100 g) was added to a round bottom flask. Pluronic 17R2 (100 g) from BASF and Genapol X-050 (200 g) from Clariant were added and mixed with an overhead stirrer until homogenous.

Preparation Example 2

Hydrosilylation of 1-(2-trimethylsilylethyl)-1,1-dimethylsilane with a methyl capped allylpolyethyleneglycol, Catalysis with 5% Platinum on Alumina (1.35 Moles of APEG400 to 1 Mole carbodisilane)

To a 4-necked, 22 L round bottom flask, equipped with an overhead stirrer, Friedrich condenser, temperature sensor (thermocouple connected to a temperature controller), heating mantle and nitrogen purge tube were charged 12340.5 grams (30.9 moles) of a methyl capped allylpolyethyleneglycol (MW ~ 400 g/mole), 3834.4 grams (95.6% purity, 22.9 moles) of 1-(2-trimethylsilylethyl)-1,1-dimethylsilane and 6.4 grams of 5% platinum on alumina (20 ppm Pt based on total flask charge). Under a nitrogen blanket, agitation was initiated and the temperature of the flask contents slowly brought to 130°C. The reaction mixture was allowed to stir for 18 hours (overnight) at 130°C. After 18 hours, 0.1 cc$H_2$/g of silanic hydrogen was detected in the product. The Friedrich condenser was removed and the flask was fitted with a cold-finger condenser and a receiver and a nitrogen sparge was initiated. After 60 minutes, the product was permitted to cool to room temperature and subsequently filtered under nitrogen pressure through a stainless steel pressure filter fitted with a 5-micron filter pad to yield 15830.0 grams of a clear, colorless fluid (Structure 2).

Structure 2

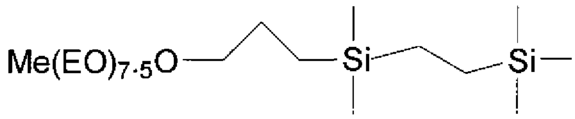

Preparation Example 2b

The product of Preparation Example 2 (100 g) was added to a round bottom flask. Pluronic 17R2 (100 g) from BASF and Genapol X-050 (200 g) from Clariant were added and mixed with an overhead stirrer until homogenous.

Preparation Example 3

Hydrosilylation of 1-(2-trimethylsilylethyl)-1,1-dimethylsilane with a methyl capped allylpolyethyleneglycol, Catalysis with 3.6% Platinum on Silica (1.35 Moles of APEG400 to 1 Mole carbodisilane)

To a 4-necked, 500 mL round bottom flask, equipped with an overhead stirrer, Friedrich condenser, temperature sensor (thermocouple connected to a temperature controller), heating mantle and nitrogen purge tube were charged 380.9 grams (0.952 moles) of a methyl capped allylpolyethyleneglycol (MW ~ 400 g/mole), 119.3 grams (94.6% purity, 0.704 moles) of 1-(2-trimethylsilylethyl)-1,1-dimethylsilane and 0.28 grams of 3.6% platinum on silica (20 ppm Pt based on total flask charge). Under a nitrogen blanket, agitation was initiated and the temperature of the flask contents slowly brought to 130°C. The reaction mixture was allowed to stir for 23 hours (overnight) at 130°C. After 18 hours, 0.4 $ccH_2/g$ of silanic hydrogen was detected in the product. The Friedrich condenser was removed and the flask was fitted with a cold-finger condenser and a receiver and a nitrogen sparge was initiated. After 60 minutes, the product was permitted to cool to room temperature and subsequently filtered under nitrogen pressure through a stainless steel pressure filter fitted with a 5-micron filter pad to yield a clear, pale yellow fluid (Structure 3).

Structure 3

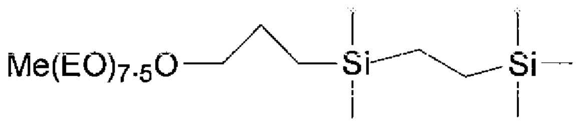

Preparation Example 3b

The product of Preparation Example 3 (100 g) was added to a round bottom flask. Pluronic 17R2 (100 g) from BASF and Genapol X-050 (200 g) from Clariant were added and mixed with an overhead stirrer until homogenous.

Comparative Example 1 CPA catalyzed hydrosilylation

A 200 mL round bottom flask was equipped with a magnetic stir bar, reflux condenser with $N_2$ inlet, thermocouple, addition funnel and heating mantle.

Allylpolyethyleneglycol (MW ~ 400 g/mole) (33.7 g; 0.0810 mol), chloroplatinic acid (10 ppm) and sodium propionate (50 mg) were charged to the round bottom flask, stirred and brought to 80 °C. The solution of 1-(2-trimethylsilylethyl)-1,1-dimethylsilane in toluene (45.5g of 22% solution; 10.0g, 0.0623 mol Y-17223) was charged to the addition funnel and added drop wise to the flask. Minor exotherm noted, addition continued over 45 min. After complete addition, the reaction was maintained at 85 °C for 1h. Reaction sample tested for Si-H content, found 0 cc H2/ g remaining. The mixture was stripped (~10 mm Hg, 100 °C) for 1.5h to remove toluene, allowed to cool to <40 °C, treated with Celite and sodium bicarbonate, stirred, pressure filtered, and bottled.

Preparation Example 4

The product of comparative example 1 (190 g) was passed through a heated jacked column that contained mercapto-functional silane (25 mL) MVE-1 from Phosphonics. The residence time was controlled by a stopcock located at the bottom of the column. The temperature was maintained at 90 °C and the residence time was 0.44 hr. After the treatment the residual Pt level fell from 9.2 ppm to 0.17 ppm.

Application Example 1 ($H_2O_2$) pressure test

To test the stability of the hydrogen peroxide in the presence of the surfactants of this invention a pressure test was conducted. As the hydrogen peroxide decomposes oxygen is liberated. The rate of oxygen liberation was tested as follows. Shown in Figure 1 is a picture of the set up. A pressure transducer cell is mounted on a pressure vessel that contains a peroxide formulation and a magnetic stirrer. The pressure vessel placed on top of a magnetic stirrer inside an oven. The pressure is monitored overtime, typically every 5 minutes for a 24 hr period. In Figure 1, (1) is the pressure transducer, (2) is the pressure vessel and (3) is the magnetic stirrer.

Formulations

TABLE 1

| | Control Formulation | Formulation Example 1 | Formulation Example 2 | Formulation Example 3 | Formulation Example 4 | Formulation Example 5 | Formulation Example 6 |
|---|---|---|---|---|---|---|---|
| Buffer | 99.25% | 99.22% pH 3 | 99.22% pH 5 | 99.22% pH 7 | 99.22% pH 10 | 99.22% pH 5 | 99.22% pH 5 |
| Hydrogen Peroxide 30% Solution | 0.75% | 0.75% | 0.75% | 0.75% | 0.75% | 0.75% | 0.75% |
| Preparation Example 1 | | 0.03% | 0.03% | 0.03% | 0.03% | | |
| Preparation Example 4 | | | | | | 0.03% | |
| Comparative Example 1 | | | | | | | 0.03% |

Results TABLE 2

| Formulation | ΔP at 10 Hr (psi) |
|---|---|
| Formulation Example 1 | 2.3 |
| Formulation Example 2 | 2.1 |
| Formulation Example 3 | 2.8 |
| Formulation Example 4 | 2.7 |
| Formulation Example 5 | 2.1 |
| Comparative Formulation Example 1 | 5.8 |

All the formulations except Comparative Example 1 did not develop excess gas The initial pressure (2-3 psi) build is from heating the closed system to 50 °C.

Application Example 2 Sunless Tanner Application

Formulation

TABLE 3

| INGREDIENT | FUNCTION | CONTROL | TEST |
|---|---|---|---|
| water | | 49.10% | 48.60% |
| propylene glycol | Humectant | 5.00% | 5.00% |
| Xanthan gum | Thickener/structuring agent | 0.75% | 0.75% |
| PEG-40 stearate | emulsifier | 0.40% | 0.40% |
| Steareth-2 | emulsifier | 0.70% | 0.70% |
| Cetearyl alcohol (and) ceteareth-20 | Emulsifier | 1.00% | 1.00% |
| Cetyl Alcohol | emulsifier | 1.50% | 1.50% |
| Glyceryl Stearate (and) PEG-100 stearate | emulsifier | 1.20% | 1.20% |
| Isononyl isononanoate | Aesthetic modifier | 28.50% | 28.50% |
| Cyclomethicone | Aesthetic modifier | 5.00% | 5.00% |
| Dimethicone, 100 cst | Aesthetic modifier | 0.50% | 0.50% |
| Preparation Example 1b | Super spreader | X | 0.50% |
| Citric acid, 50% solution | PH adjuster | 0.05% | 0.05% |
| DHA | Self-tanning active | 5.00% | 5.00% |
| Phenoxyethanol and Paraben blend | Preservative | 1.30% | 1.30% |

1 gram of product was applied to a 1.5 $inch^2$ area on the forearm of test subject with Fitzpatrick's type II skin coloring. The mechanism of dihydroxyacetone in a self-tanner can be describes as the Maillard reaction. The DHA reacts with the amino acids in the skin to form a melanin-like compound known as melanoidins. This reaction was measured using ImageJ imaging software. The procedure for this measure is as follows: convert image from color to gray scale. The intensity of the color of each pixel is measured from left to right and plotted. The plot indicates the intensity of the pixels on the Y axis form a range of 256 (being white) to zero being black. The lower number on the Y-axis is indicative of more pigmented skin. The X axis represents the length of the photograph, where 300 -1200 on the x axis represents the test product and 1700 to 2600 on the x axis represents the control product. The plot is shown in Figure 2 and Figure 3.

A picture was taken at a 3-hour time-point and color intensity was analyzed and plotted in Figure 2. When

Preparation Example 1 was added to a DHA containing self-tanner, it accelerated the Maillard reaction in the skin and the melanoidins were able to form about 25% quicker, which was expressed in color development at 3 hours as opposed to 4 hours in the control.

At a 24-hour time period, pictures of the same test site were taken and analyzed in the same manner. The pigment intensity results are shown in Figure 3. It was observed after 24 hours and the color fully developed that the product containing Preparation Example 1b showed a more prominent color distribution than the control where the color was very uneven.

Application Example 3 (Hair Straightening)

Example Formulation of waving lotion

TABLE 4

| Ingredients | Application Example 3a (wt%) | Comparative Example 3a (wt%) |
|---|---|---|
| Ammonium Thioglycolate | 7 | 7 |
| Potassium hydroxide | 1 | 1 |
| Disodium EDTA | 0.5 | 0.5 |
| Preparation Example 1b | 0.5 | 0 |
| water | q.s. to 100 | q.s to 100 |

pH was adjusted to 9.3

Example of neutralizing and oxidizing lotion for waving hair

TABLE 5

| Ingredient | Application Example 3b (wt%) | Comparative Example 3b (wt %) |
|---|---|---|
| Hydrogen peroxide | 2 | 2 |
| Preparation Example 1b | 0.5 | 0 |
| water | q.s to 100 | q.s to 100 |

pH=6

Protocol: The experiment was conducted at 40 °C in a water bath. Twenty hair fibers (virgin brown hair from International Hair Importers) were coiled on a 5 mm diameter glass rod and held with two O-rings. The glass rod was immersed in 10 ml of the waving solution for 2 min in a test tube (Application Example 3a or Comparative Example 3b). The hair was rinsed for 15 seconds in clean water, and then immersed in the oxidizing solution for 15 min (Application Example 3b or Comparative Example 3b). The hair was rinsed 15 seconds in clean water and dried at 45 °C. The hair coil was removed from the rod. The length of the coil was measured ($L_o$). The hair coil was placed in a humidity chamber at 90% RH for 1 h. The length of the coil was measured after the humidity chamber ($L_f$).

The hair treated with the waving lotion containing Application Example 3a and the oxidizing solution with Application Example 3b showed a better curl retention than the negative control (Comparative Example 3a and 3b).

TABLE 6

| Treatments | $L_o$ (cm) | $L_f$ (cm) |
|---|---|---|
| Negative control (hair treated with comparative 1 and 2) | 5.5 | 8.5 |
| Example (hair treated with example 1 and 2) | 5 | 7.5 |

Formulation Examples

Example 3c: Thiol permanent wave waving lotion

TABLE 7

| Ingredient | Wt% |
|---|---|
| Thioglycolic acid | 6 |
| Steareth-20 | 2.5 |
| fragrance | 0.5 |
| Ethylene diamine tetraacetic acid | 0.2 |
| Preparation Example 3b | 0.5 |
| water | q.s. to 100 |

Example 3d: Neutralizer

TABLE 8

| Ingredient | Wt% |
|---|---|
| Hydrogen peroxide (30%) | 7 |
| Polysorbate-40 | 2.5 |
| Preparation Example 2b | 0.5 |
| Phosphoric acid (85%) | To pH 4 |
| water | q.s. to 100 |

Example 3e: Waving lotion

TABLE 9

| Ingredient | Wt% |
|---|---|
| Ammonium bisulfite | 4 |
| Ammonium sulfite | 3 |
| Laurteth-23 | 2.5 |
| Preparation Example 1b | 1 |
| Ammonium hydroxide | to pH 8 |
| water | q.s. to 100 |

Application Example 5 (Hair Dye)

Six boxes of L'Oreal Feria 66 (very rich auburn) with the same lot number (HD085) were obtained for the testing. Hair tresses (2 g, 6 in, natural white) were purchased from International Hair Importers. Hair tresses were split approximately in half with one side of the tresses serving as a control for the other (test) side. Preparation Example 2b was added to the shimmering color phase at the following wt/wt concentrations: 0.05%, 0.10%, 0.25%, 0.50% and 1.00%. The hair tresses were treated and rinsed per the manufacturer's directions (25 min residence time, 2 min rinse with 42°C

tap water, +/-2°C). Hair tresses were then conditioned with the supplied conditioner and again rinsed (2 min rinse with 42°C tap water, +/- 2°C). Tresses were allowed to dry overnight at room temperature, then were brushed. The color was measured using LAB readings via a Hunter Lab Color Quest XE Spectrophotometer and recorded. ΔE was calculated taking the square root of the sum of the squares of ΔL, ΔA and ΔB. ΔL = L(with Prep. Ex 2b) - L(without Prep. Ex 2b). ΔA = A(with Prep. Ex 2b) - A(without Prep. Ex 2b). ΔB = B(with Prep. Ex 2b) - B(without Prep. The larger ΔE is, the more colored the hair tress is with Preparation Example 2B included as compared to the control.

Results:

TABLE 10

| Concentration | ΔE |
|---|---|
| 0.05% | 1.59 |
| 0.10% | 1.05 |
| 0.25% | 2.14 |
| 0.50% | 2.26 |
| 1.00% | 6.25 |

More specifically, each treated tress treated with hair dye plus Preparation Example 2b was darker than its control tress as measure by ΔL* and ΔA*;

TABLE 11

| **Concentration** | **ΔL*** |
|---|---|
| 0.05% | -0.80 |
| 0.10% | -0.99 |
| 0.25% | -1.77 |
| 0.50% | -1.13 |
| 1.00% | -4.81 |

Application Example 6 (Window Defogger)

Equipment:

2"X3.5" mirror attached to articulated arm.
Hot plate, stirrer, 2000 ml glass beaker
1 oz spray bottles
Cheesecloth

Procedure:

A 0.5% mixture of Preparation Example 1 in commercially available Windex was put into a spray bottle. A mirror, previously cleaned with isopropanol, had a line drawn with magic maker separating into two sections. The left side of the mirror was cleaned with unmodified Windex. 0.3 g which was sprayed on and wiped off with clean cheesecloth, using 5 up and down movements. The right side was protected during spraying with Teflon tape and a piece of hard plastic. The right side of the mirror was treated in the same fashion using the Windex with 0.5% Preparation Example 1 added. The mirrors were allowed to dry for 15 minutes. The mirrors were then placed 2 inches above the boiling water beaker. The results were photographed

Results:

Both mirrors appeared clear after application. The steam vapor coalesced on the surface of the mirror. The water droplets did not bead on the right side of the mirror treated with Windex with Preparation Example 1 thus reducing a fogged appearance, but did bead on the left side which was only treated with Windex. This is shown in FIGURE 4.

## Claims

**1.** A personal care or home care composition comprising:

(a) a silicon containing compound having the formula:

$$(R^1)(R^2)(R^3)Si\text{-}R^4\text{-}Si(R^5)(R^6)(R^7)$$

wherein

$R^1$, $R^2$, $R^3$, $R^5$, and $R^6$ are each independently selected from the group consisting of 1 to 6 monovalent hydrocarbon radicals, aryl, and a hydrocarbon group of 7 to 10 carbons containing an aryl group;
$R^4$ is a hydrocarbon group of 1 to 3 carbons;
$R^7$ is an alkyleneoxide group of the general formula:

$$R^8(C_2H_4O)_d(C_3H_6O)_e(C_4H_8O)_fR^9$$

where

$R^8$ is a divalent linear or branched hydrocarbon radical having the structure:

$$\text{-}CH_2\text{-}CH(R^{10})(R^{11})_gO\text{-}$$

where $R^{10}$ is H or methyl;
$R^{11}$ is a divalent alkyl radical of 1 to 6 carbons where the subscript g is 0 or 1;
$R^9$ is selected from the group consisting of H, monovalent hydrocarbon radicals of 1 to 6 carbon atoms and acetyl, subject to the limitation that the subscripts e and f are zero or positive and satisfy the following relationships:

$$2 \leq d + e + f \leq 20 \text{ with } d \geq 2;$$

and

b) an personal care or home care active component which is a peroxide, wherein the composition contains less than 2 ppm of catalyst.

**2.** The composition of claim 1 where the peroxide is one selected from the group consisting of hydrogen peroxide, ammonium persulfate, potassium persulfate, diisopropyl peroxide, dilauryl peroxide; di-t-butyl peroxide, dicumyl peroxide, t-butyl hydrogen peroxide, t-amyl hydrogen peroxide, cumyl hydrogen peroxide, acetyl peroxide, lauroyl peroxide, benzoyl peroxide, ethyl peroxybenzoate and pavalate peroxide, as well as peroxides of, for instance, lipids, proteins, carbohydrates and free radicals derived from the same.

**3.** The composition of claim 1 where the amount of peroxide is from 0.1 weight percent to 10 weight percent, more specifically from 1 to 7 weight percent based on the weight of the composition.

**4.** The composition of claim 1 where $R^1$, $R^2$, $R^3$, $R^5$, and $R^6$ are methyl.

**5.** The composition of claim 4 where $R^{10}$ is hydrogen or wherein $R^{10}$ is methyl.

**6.** The composition of claim 5 where the subscript g is zero.

**7.** The composition of claim 5 where the subscript g is one.

**8.** The composition of claim 7 where $R^{11}$ is $-CH_2-$.

**9.** The composition of claim 7 where $R^{11}$ is a divalent alkyl radical of 2 carbons.

**10.** An aqueous emulsion wherein the discontinuous phase comprises water and the continuous phase comprises the composition of claim 1.

**11.** An aqueous emulsion wherein the continuous phase comprises water and the discontinuous phase comprises the composition of claim 1.

**12.** A non-aqueous emulsion wherein the discontinuous phase comprises a non-aqueous hydroxylic solvent and the continuous phase comprises the composition of claim 1.

**13.** A non-aqueous emulsion wherein the continuous phase comprises a non-aqueous hydroxylic solvent and the discontinuous phase comprises the composition of claim 1.

**14.** A method of making a silicon containing compound having the formula:

$$(R^1)(R^2)(R^3)Si\ -R^4-Si(R^5)(R^6)(R^7)$$

wherein

$R^1$, $R^2$, $R^3$, $R^5$, and $R^6$ are each independently selected from the group consisting of 1 to 6 monovalent hydrocarbon radicals, aryl, and a hydrocarbon group of 7 to 10 carbons containing an aryl group;
$R^4$ is a hydrocarbon group of 1 to 3 carbons;
$R^7$ is an alkyleneoxide group of the general formula:

$$R^8(C_2H_4O)_d(C_3H_6O)_e(C_4H_8O)_fR^9$$

where

$R^8$ is a divalent linear or branched hydrocarbon radical having the structure:

$$-CH_2-CH(R^{10})(R^{11})_gO-$$

where $R^{10}$ is H or methyl;
$R^{11}$ is a divalent alkyl radical of 1 to 6 carbons where the subscript g is 0 or 1;
$R^9$ is selected from the group consisting of H, monovalent hydrocarbon radicals of 1 to 6 carbon atoms and acetyl,

subject to the limitation that the subscripts e and f are zero or positive and satisfy the following relationships:

$$2 \leq d + e + f \leq 20 \text{ with } d \geq 2;$$

and comprising:
reacting a molecule of the following formula:

$$(R^1)(R^2)(R^3)Si\ -R^4-Si(R^5)(R^6)(R^{12})$$

wherein $R^1$-$R^6$ are as defined and where $R^{12}$ is H, under hydrosilylation conditions in the presence of a heterogenous supported catalyst, with an olefinically modified polyalkyleneoxide, wherein the amount of catalyst in the reaction mixture is reduced after the reaction to an amount of less than 2 ppm based on the weight of the silicon containing compound having the formula $(R^1)(R^2)(R^3)Si-R^4-Si(R^5)(R^6)(R^7)$.

**15.** The method of Claim 14 wherein the polyether is an olefinically modified polyalkyleneoxide described by the general formula:

$$CH_2{=}CH(R^{10})(R^{11})_gO(C_2H_4O)_d(C_3H_6O)_e(C_4H_8O)_fR^9$$

where
$R^{10}$ is H or methyl; $R^{11}$ is a divalent alkyl radical of 1 to 6 carbons where the subscript g may be 0 or 1; $R^9$ is H, a monofunctional hydrocarbon radical of 1 to 6 carbons, or acetyl.

## Patentansprüche

**1.** Körperpflege- oder Hauspflegezusammensetzung, umfassend:

(a) eine Silicium enthaltende Verbindung mit der Formel:

$$(R^1)(R^2)(R^3)Si\text{ -}R^4\text{-}Si(R^5)(R^6)(R^7)$$

wobei

$R^1$, $R^2$, $R^3$, $R^5$ und $R^6$ jeweils unabhängig gewählt sind aus der Gruppe bestehend aus 1 bis 6 monovalenten Kohlenwasserstoffradikalen, Aryl und einer Kohlenwasserstoffgruppe mit 7 bis 10 Kohlenstoffen, die eine Arylgruppe enthält;
$R^4$ eine Kohlenwasserstoffgruppe mit 1 bis 3 Kohlenstoffen ist;
$R^7$ eine Alkylenoxidgruppe der allgemeinen Formel:

$$R^8(C_2H_4O)_d(C_3H_6O)_e(C_4H_8O)_fR^9$$

ist,
wobei
$R^8$ ein bivalentes lineares oder verzweigtes Kohlenwasserstoffradikal mit der Struktur:

$$-CH_2\text{-}CH(R^{10})(R^{11})_gO\text{-}$$

ist,

wobei $R^{10}$ H oder Methyl ist;
$R^{11}$ ein bivalentes Alkylradikal mit 1 bis 6 Kohlenstoffen ist, wobei die Tieferstellung g 0 oder 1 ist;
$R^9$ gewählt ist aus der Gruppe bestehend aus H, monovalenten Kohlenwasserstoffradikalen mit 1 bis 6 Kohlenstoffatomen und Acetyl,

vorbehaltlich der Einschränkung, dass die Tieferstellungen e und f null oder positiv sind und die folgenden Beziehungen erfüllen:

$$2 \leq d + e + f \leq 20, \text{ wobei } d \geq 2;$$

und
b) eine Körperpflege- oder Hauspflege-Wirkkomponente, die ein Peroxid ist,

wobei die Zusammensetzung weniger als 2 ppm Katalysator enthält.

**2.** Zusammensetzung nach Anspruch 1, wobei das Peroxid eines gewählt aus der Gruppe bestehend aus Wasserstoffperoxid, Ammoniumpersulfat, Kaliumpersulfat, Diisopropylperoxid, Dilaurylperoxid, Di-t-butylperoxid, Dicumylperoxid, t-Butylwasserstoffperoxid, t-Amylwasserstoffperoxid, Cumylwasserstoffperoxid, Acetylperoxid, Lauroylperoxid, Benzoylperoxid, Ethylperoxybenzoat und Pavalatperoxid sowie Peroxiden von beispielsweise Lipiden, Proteinen, Kohlenhydraten und freien Radikalen, die aus diesen hervorgehen, ist.

3. Zusammensetzung nach Anspruch 1, wobei die Peroxidmenge beruhend auf dem Gewicht der Zusammensetzung 0,1 Gewichtsprozent bis 10 Gewichtsprozent, insbesondere 1 bis 7 Gewichtsprozent, beträgt.

4. Zusammensetzung nach Anspruch 1, wobei $R^1$, $R^2$, $R^3$ , $R^5$ und $R^6$ Methyl sind.

5. Zusammensetzung nach Anspruch 4, wobei $R^{10}$ Wasserstoff ist oder wobei $R^{10}$ Methyl ist.

6. Zusammensetzung nach Anspruch 5, wobei die Tieferstellung g null ist.

7. Zusammensetzung nach Anspruch 5, wobei die Tieferstellung g eins ist.

8. Zusammensetzung nach Anspruch 7, wobei $R^{11}$ $-CH_2-$ ist.

9. Zusammensetzung nach Anspruch 7, wobei $R^{11}$ ein bivalentes Alkylradikal mit 2 Kohlenstoffen ist.

10. Wässrige Emulsion, wobei die diskontinuierliche Phase Wasser umfasst und die kontinuierliche Phase die Zusammensetzung nach Anspruch 1 umfasst.

11. Wässrige Emulsion, wobei die kontinuierliche Phase Wasser umfasst und die diskontinuierliche Phase die Zusammensetzung nach Anspruch 1 umfasst.

12. Nichtwässrige Emulsion, wobei die diskontinuierliche Phase ein nichtwässriges hydroxylisches Lösungsmittel umfasst und die kontinuierliche Phase die Zusammensetzung nach Anspruch 1 umfasst.

13. Nichtwässrige Emulsion, wobei die kontinuierliche Phase ein nichtwässriges hydroxylisches Lösungsmittel umfasst und die diskontinuierliche Phase die Zusammensetzung nach Anspruch 1 umfasst 1.

14. Verfahren zum Herstellen einer siliciumhaltigen Verbindung mit der Formel:

$$(R^1)(R^2)(R^3)Si\text{ -}R^4\text{-}Si(R^5)(R^6)(R^7)$$

wobei

$R^1$, $R^2$, $R^3$, $R^5$ und $R^6$ jeweils unabhängig aus der Gruppe bestehend aus 1 bis 6 monovalenten Kohlenwasserstoffradikalen, Aryl und einer Kohlenwasserstoffgruppe mit 7 bis 10 Kohlenstoffen, die eine Arylgruppe enthält, gewählt sind;
$R^4$ eine Kohlenwasserstoffgruppe mit 1 bis 3 Kohlenstoffen ist;
$R^7$ eine Alkylenoxidgruppe der allgemeinen Formel:

$$R^8(C_2H_4O)_d(C_3H_6O)_e(C_4H_8O)_fR^9$$

ist,
wobei
$R^8$ ein bivalentes lineares oder verzweigtes Kohlenwasserstoffradikal mit der Struktur:

$$-CH_2\text{-}CH(R^{10})(R^{11})gO\text{-}$$

ist,

wobei $R^{10}$ H oder Methyl ist;
$R^{11}$ ein bivalentes Alkylradikal mit 1 bis 6 Kohlenstoffen ist, wobei die Tieferstellung g 0 oder 1 ist;
$R^9$ aus der Gruppe bestehend aus H, monovalenten Kohlenwasserstoffradikalen mit 1 bis 6 Kohlenstoffatomen und Acetyl gewählt ist,

vorbehaltlich der Einschränkung, dass die Tieferstellungen e und f null oder positiv sind und die folgenden Beziehungen erfüllen:

$$2 \leq d + e + f \leq 20, \text{ wobei } d \geq 2;$$

und umfassend:
Reagieren eines Moleküls der folgenden Formel:

$$(R^1)(R^2)(R^3)Si\ R^4\text{-}S_i(R^5)(R^6)(R^{12})$$

wobei $R^1$-$R^6$ wie definiert sind und wobei $R^{12}$ H ist, unter Hydrosilylierungsbedingungen bei Vorhandensein eines heterogenen geträgerten Katalysators, mit einem olefinisch modifiziertem Polyalkylenoxid, wobei die Katalysatormenge in dem Reaktionsgemisch nach der Reaktion beruhend auf dem Gewicht der siliciumhaltigen Verbindung mit der Formel $(R^1)(R^2)(R^3)Si\text{-}R^4\text{-}Si(R^5)(R^6)(R^7)$ auf eine Menge von weniger als 2 ppm reduziert ist.

**15.** Verfahren nach Anspruch 14, wobei der Polyether ein olefinisch modifiziertes Polyalkylenoxid ist, welches durch die allgemeine Formel:

$$CH_2{=}CH(R^{10})(R^{11})_gO(C_2H_4O)_d(C_3H_6O)_e(C_4H_8O)_fR^9$$

beschrieben ist,
wobei
$R^{10}$ H oder Methyl ist; $R^{11}$ ein bivalentes Alkylradikal mit 1 bis 6 Kohlenstoffen ist, wobei die Tieferstellung g 0 oder 1 sein kann; $R^9$ H, ein monofunktionelles Kohlenwasserstoffradikal mit 1 bis 6 Kohlenstoffen oder Acetyl ist.

## Revendications

**1.** Composition de soin personnel ou d'entretien de la maison comprenant :

(a) un composé contenant du silicium ayant la formule :

$$(R^1)(R^2)(R^3)Si\ \text{-}R^4\text{-}Si(R^5)(R^6)(R^7)$$

dans laquelle

$R^1$, $R^2$, $R^3$, $R^5$ et $R^6$ sont choisis chacun indépendamment dans le groupe constitué de 1 à 6 radicaux hydrocarbures monovalents, d'aryle et d'un groupe hydrocarbure de 7 à 10 carbones contenant un groupe aryle ;
$R^4$ est un groupe hydrocarbure de 1 à 3 carbones ;
$R^7$ est un groupe oxyde d'alkylène de formule générale :

$$R^8(C_2H_4O)_d(C_3H_6O)_e(C_4H_8O)_fR^9$$

où
$R^8$ est un radical hydrocarbure divalent linéaire ou ramifié ayant la structure :

$$\text{-}CH_2\text{-}CH(R^{10})(R^{11})_gO\text{-}$$

où

$R^{10}$ est H ou un méthyle ;
$R^{11}$ est un radical alkyle divalent de 1 à 6 carbones où l'indice g vaut 0 ou 1 ;
$R^9$ est choisi dans le groupe constitué de H, de radicaux hydrocarbures monovalents de 1 à 6 atomes de carbone et d'acétyle,

à condition que les indices e et f soient positifs ou nuls et qu'ils satisfassent les relations suivantes :

$$2 \leq d + e + f \leq 20 \text{ avec } d \geq 2 ;$$

et

b) un constituant actif de soin personnel ou d'entretien de la maison qui est un peroxyde,

la composition contenant moins de 2 ppm de catalyseur.

**2.** Composition selon la revendication 1 où le peroxyde est l'un choisi dans le groupe constitué de peroxyde d'hydrogène, persulfate d'ammonium, persulfate de potassium, peroxyde de diisopropyle, peroxyde de dilauryle ; peroxyde de di-*t*-butyle, peroxyde de dicumyle, hydrogénoperoxyde de *t*-butyle, hydrogénoperoxyde de *t*-amyle, hydrogénoperoxyde de cumyle, peroxyde d'acétyle, peroxyde de lauroyle, peroxyde de benzoyle, peroxybenzoate d'éthyle et peroxyde de pavalate, ainsi que des peroxydes, par exemple, de lipides, de protéines, de glucides et des radicaux libres dérivés de ceux-ci.

**3.** Composition selon la revendication 1 où la quantité de peroxyde va de 0,1 pourcent en poids à 10 pourcent en poids, plus spécifiquement de 1 à 7 pourcent en poids sur la base du poids de la composition.

**4.** Composition selon la revendication 1 où $R^1$, $R^2$, $R^3$, $R^5$ et $R^6$ sont un méthyle.

**5.** Composition selon la revendication 4 où $R^{10}$ est l'hydrogène ou dans laquelle $R^{10}$ est un méthyle.

**6.** Composition selon la revendication 5 où l'indice g vaut zéro.

**7.** Composition selon la revendication 5 où l'indice g vaut un.

**8.** Composition selon la revendication 7 où $R^{11}$ est $-CH_2-$.

**9.** Composition selon la revendication 7 où $R^{11}$ est un radical alkyle divalent de 2 carbones.

**10.** Émulsion aqueuse dans laquelle la phase discontinue comprend de l'eau et la phase continue comprend la composition selon la revendication 1.

**11.** Émulsion aqueuse dans laquelle la phase continue comprend de l'eau et la phase discontinue comprend la composition selon la revendication 1.

**12.** Émulsion non aqueuse dans laquelle la phase discontinue comprend un solvant hydroxylique non aqueux et la phase continue comprend la composition selon la revendication 1.

**13.** Émulsion non aqueuse dans laquelle la phase continue comprend un solvant hydroxylique non aqueux et la phase discontinue comprend la composition selon la revendication 1.

**14.** Procédé de fabrication d'un composé contenant du silicium ayant la formule :

$$(R^1)(R^2)(R^3)Si\text{ -}R^4\text{-}Si(R^5)(R^6)(R^7)$$

dans laquelle

$R^1$, $R^2$, $R^3$, $R^5$ et $R^6$ sont choisis chacun indépendamment dans le groupe constitué de 1 à 6 radicaux hydrocarbures monovalents, d'aryle et d'un groupe hydrocarbure de 7 à 10 carbones contenant un groupe aryle ;
$R^4$ est un groupe hydrocarbure de 1 à 3 carbones ;
$R^7$ est un groupe oxyde d'alkylène de formule générale :

$$R^8(C_2H_4O)_d(C_3H_6O)_e(C_4H_8O)_fR^9$$

où $R^8$ est un radical hydrocarbure divalent linéaire ou ramifié ayant la structure :

$$-CH_2-CH(R^{10})(R^{11})_gO-$$

où

$R^{10}$ est H ou un méthyle ;
$R^{11}$ est un radical alkyle divalent de 1 à 6 carbones où l'indice g vaut 0 ou 1 ;
$R^9$ est choisi dans le groupe constitué de H, de radicaux hydrocarbures monovalents de 1 à 6 atomes de carbone et d'acétyle, à condition que les indices e et f soient positifs ou nuls et qu'ils satisfassent les relations suivantes :

$$2 \leq d + e + f \leq 20 \text{ avec } d \geq 2 \text{ ;}$$

et

comprenant
la réaction d'une molécule de formule suivante :

$$(R^1)(R^2)(R^3)Si\ R^4-Si(R^5)(R^6)(R^{12})$$

dans laquelle
$R^1$ à $R^6$ sont tels que définis et où $R^{12}$ est H, dans des conditions d'hydrosilylation en présence d'un catalyseur supporté hétérogène, avec un poly(oxyde d'alkylène) modifié par une oléfine, où
la quantité de catalyseur dans le mélange réactionnel est réduite après la réaction à une quantité inférieure à 2 ppm sur la base du poids du composé contenant du silicium ayant la formule $(R^1)(R^2)(R^3)Si-R^4-Si(R^5)(R^6)(R^7)$.

**15.** Procédé selon la revendication 14 dans lequel
le polyéther est un poly(oxyde d'alkylène) modifié par une oléfine décrit par la formule générale :

$$CH_2=CH(R^{10})(R^{11})_gO(C_2H_4O)_d(C_3H_6O)_e(C_4H_8O)_fR^9$$

où

$R^{10}$ est H ou un méthyle ;
$R^{11}$ est un radical alkyle divalent de 1 à 6 carbones où l'indice g peut valoir 0 ou 1 ;
$R^9$ est H, un radical hydrocarbure monofonctionnel de 1 à 6 carbones, ou un acétyle.

Figure 1

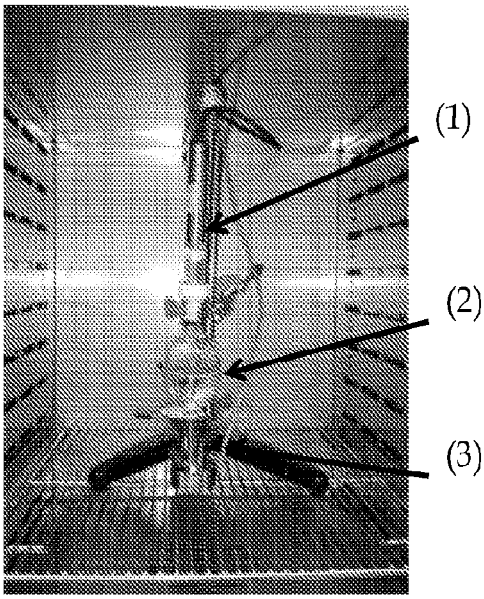

Figure 2

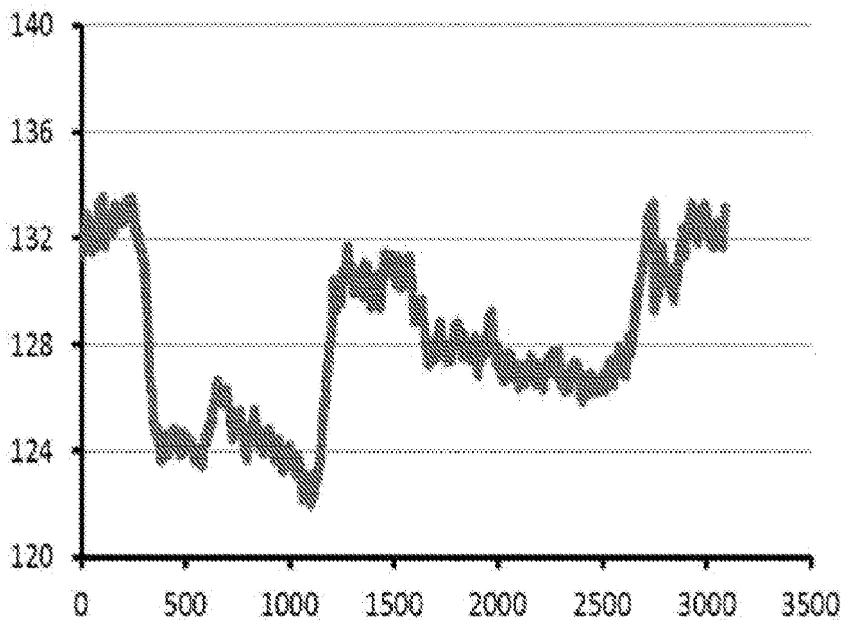

Figure 3

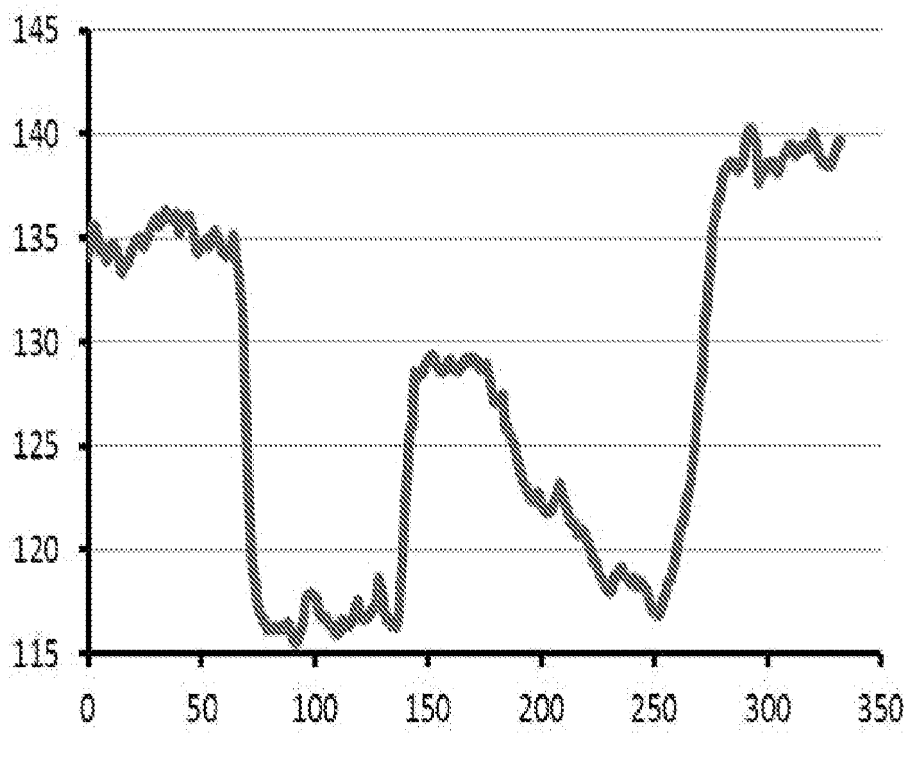

Figure 4

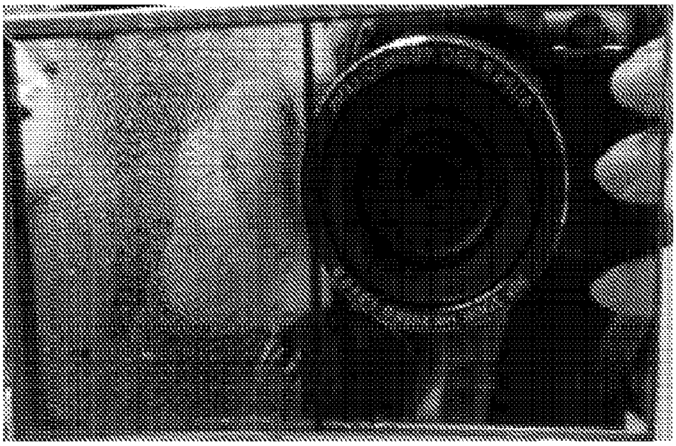

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2007127016 A1
- US 3159601 A
- US 3220972 A
- US 3715334 A
- US 3775452 A
- US 3814730 A, Karstedt
- US 8497338 B2
- US 6060546 A
- US 6271295 B
- US 6046156 A
- US 6054547 A
- US 6075111 A
- US 6077923 A
- US 6083901 A
- US 6153578 A